# EUROPEAN PATENT SPECIFICATION

(11) **EP 4 712 021 B1**
(45) Date of publication and mention of the grant of the patent: **04.11.2026**
(21) Application number: 25197864.9
(22) Date of filing: 25.08.2025
(51) Int. Cl.: G06T 7/10

(54) **ULTRASOUND DIAGNOSTIC APPARATUS AND METHOD OF CONTROLLING ULTRASOUND DIAGNOSTIC APPARATUS**
ULTRASCHALLDIAGNOSEVORRICHTUNG UND VERFAHREN ZUR STEUERUNG DER ULTRASCHALLDIAGNOSEVORRICHTUNG
APPAREIL DE DIAGNOSTIC À ULTRASONS ET PROCÉDÉ DE COMMANDE D'APPAREIL DE DIAGNOSTIC À ULTRASONS

(30) Priority: 11.09.2024 JP 2024157286
(43) Date of publication of application: 18.03.2026
(73) Proprietor: FUJIFILM Corporation, Tokyo 106-8620 (JP)
(72) Inventor: RAGHAVA, Krishnan, Kaisei (JP)
(74) Representative: Meissner Bolte Partnerschaft mbB

(56) References cited:
- GB-A- 2 611 602
- JP-A- 2012 081 177
- US-B2- 11 763 460
- US-B2- 12 002 216

## Description

### BACKGROUND OF THE INVENTION

### 1. Field of the Invention

The present invention relates to an ultrasound diagnostic apparatus that captures an ultrasound image of a heart of a subject and a control method of the ultrasound diagnostic apparatus.

### 2. Description of the Related Art

In the related art, it is known that an ultrasound image representing a tomographic plane of a heart of a subject is captured using a so-called ultrasound diagnostic apparatus, and an ejection fraction of a lumen of the heart, such as a so-called left ventricular ejection fraction (LVEF), is calculated using the ultrasound image. In a case of calculating the ejection fraction of the lumen of the heart, an ultrasound image representing end-systolic and end-diastolic states of the heart in a so-called apical two-chamber cross section and an ultrasound image representing end-systolic and end-diastolic states of the heart in a so-called apical four-chamber cross section are often used.

In addition, in a case of calculating the ejection fraction of the lumen of the heart, for example, the contour of the lumen of the heart is automatically extracted by performing so-called segmentation on each ultrasound image, and the ejection fraction is calculated using the area surrounded by the contour in many cases. In such a series of processing, a contour different from the original contour of the lumen of the heart may be extracted as the contour of the lumen of the heart automatically extracted due to a presence of a so-called artifact appearing in the ultrasound image or the like. In order to easily correct such an error in segmentation, for example, an ultrasound diagnostic apparatus as disclosed in JP2022-172765A has been developed. The ultrasound diagnostic apparatus of JP2022-172765A has a plurality of correction modes corresponding to a plurality of correction methods of the contour of the extracted structure in the heart, and automatically corrects the contour using the correction mode selected by the user.

### SUMMARY OF THE INVENTION

However, in the technique of JP2022-172765A, in order to find an ultrasound image in which an error has occurred in segmentation, a user of the ultrasound diagnostic apparatus needs to check the ultrasound images of a plurality of frames, and thus it may require a significant amount of time to correct a segmentation result.

The present invention has been made in order to solve such a problem in the related art, and an object of the invention is to provide an ultrasound diagnostic apparatus and a control method of the ultrasound diagnostic apparatus capable of easily correcting the segmentation result of a lumen of a heart in a short time.

With the following configurations, the above-described object can be achieved.
[1] An ultrasound diagnostic apparatus comprising: an image acquisition unit that acquires ultrasound images of a plurality of frames that are continuous in time series and in which a heart of a subject is imaged; a contour extraction unit that performs segmentation on the ultrasound images of the plurality of frames to extract a contour of a lumen of the heart for each frame; a cross-sectional area calculation unit that calculates a cross-sectional area of the lumen of the heart for each frame based on the contour extracted by the contour extraction unit; a cross-sectional area change curve generation unit that generates a cross-sectional area change curve representing an actual temporal change of the cross-sectional area based on the cross-sectional area calculated by the cross-sectional area calculation unit; and an error detection unit that detects an error of the segmentation performed by the contour extraction unit by comparing the cross-sectional area change curve generated by the cross-sectional area change curve generation unit with a predicted cross-sectional area change prediction curve.
[2] The ultrasound diagnostic apparatus according to [1], in which the error detection unit notifies a user of a detection result of the error.
[3] The ultrasound diagnostic apparatus according to [1] or [2], in which the error detection unit detects that the cross-sectional area change curve and the cross-sectional area change prediction curve are at least partially separated from each other as the error.
[4] The ultrasound diagnostic apparatus according to any one of [1] to [3], further comprising: a prediction curve generation unit that generates the cross-sectional area change prediction curve based on the cross-sectional area change curve generated by the cross-sectional area change curve generation unit.
[5] The ultrasound diagnostic apparatus according to any one of [1] to [4], further comprising an error correction unit that corrects the error of the segmentation performed by the contour extraction unit.
[6] The ultrasound diagnostic apparatus according to [5], in which the error correction unit sets a plurality of control points operable by a user on the contour extracted by the contour extraction unit, and corrects the error of the segmentation by deforming the contour based on movement of at least one of the plurality of control points by the user.
[7] The ultrasound diagnostic apparatus according to [6], in which the error correction unit calculates the number of inflection points in the contour extracted by the contour extraction unit in an image region specified by the user, detects an edge of the lumen of the heart in the image region and calculates the number of inflection points in the edge, and sets the number of the plurality of control points by comparing the number of inflection points in the contour with the number of inflection points in the edge.
[8] A control method of an ultrasound diagnostic apparatus, the control method comprising: acquiring ultrasound images of a plurality of frames that are continuous in time series and in which a heart of a subject is imaged; performing segmentation on the ultrasound images of the plurality of frames to extract a contour of a lumen of the heart for each frame; calculating a cross-sectional area of the lumen of the heart for each frame based on the extracted contour; generating a cross-sectional area change curve representing an actual temporal change of the cross-sectional area based on the calculated cross-sectional area; and detecting an error of the segmentation by comparing the generated cross-sectional area change curve with a predicted cross-sectional area change prediction curve.

The present invention provides an ultrasound diagnostic apparatus comprising: an image acquisition unit that acquires ultrasound images of a plurality of frames that are continuous in time series and in which a heart of a subject is imaged; a contour extraction unit that performs segmentation on the ultrasound images of the plurality of frames to extract a contour of a lumen of the heart for each frame; a cross-sectional area calculation unit that calculates a cross-sectional area of the lumen of the heart for each frame based on the contour extracted by the contour extraction unit; a cross-sectional area change curve generation unit that generates a cross-sectional area change curve representing an actual temporal change of the cross-sectional area based on the cross-sectional area calculated by the cross-sectional area calculation unit; and an error detection unit that detects an error of the segmentation performed by the contour extraction unit by comparing the cross-sectional area change curve generated by the cross-sectional area change curve generation unit with a predicted cross-sectional area change prediction curve. Therefore, the segmentation result of the lumen of the heart can be easily corrected in a short time.

### BRIEF DESCRIPTION OF THE DRAWINGS

Fig. 1 is a block diagram showing a configuration of an ultrasound diagnostic apparatus according to an embodiment of the present invention.
Fig. 2 is a block diagram showing an internal configuration of a transmission and reception circuit in an embodiment of the present invention.
Fig. 3 is a block diagram showing an internal configuration of an image generation unit in an embodiment of the present invention.
Fig. 4 is a diagram schematically showing an example of an ultrasound image representing an apical four-chamber cross section.
Fig. 5 is a diagram showing an example of a cross-sectional area change curve of a lumen of a heart in an ultrasound image in a plurality of frames.
Fig. 6 is a diagram showing an example of a cross-sectional area change curve in which a segmentation error occurs on a contour of the lumen of the heart in some of the ultrasound images of the plurality of frames.
Fig. 7 is a diagram showing an example of a cross-sectional area change prediction curve calculated based on a cross-sectional area change curve.
Fig. 8 is a diagram showing an example of a plurality of control points in a case where a contour including more unevenness than a contour of a lumen of a heart detected by edge detection processing is extracted by segmentation.
Fig. 9 is a diagram showing an example of a plurality of control points in a case where a contour smoother than a contour of a lumen of a heart detected by edge detection processing is extracted by segmentation.
Fig. 10 is a flowchart showing an operation of the ultrasound diagnostic apparatus according to an embodiment of the present invention.

### DESCRIPTION OF THE PREFERRED EMBODIMENTS

Hereinafter, embodiments of the present invention will be described based on the accompanying drawings.

The following configuration requirements are described based on a representative embodiment of the present invention, but the present invention is not limited to such an embodiment.

It should be noted that, in the present specification, a numerical range represented by "to" means a range including numerical values described before and after "to", both ends inclusive, as a lower limit value and an upper limit value.

In the present specification, "the same" includes an error range generally allowed in the technical field.

### Embodiment

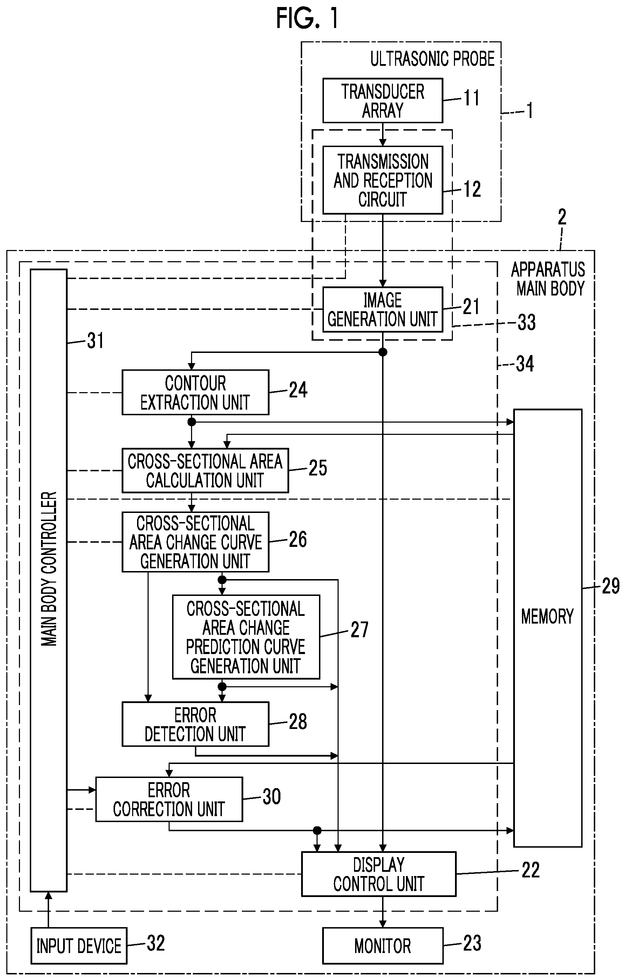

Fig. 1 shows a configuration of an ultrasound diagnostic apparatus according to an embodiment of the present invention. The ultrasound diagnostic apparatus comprises an ultrasound probe 1 and an apparatus main body 2 connected to each other by so-called wired communication or so-called wireless communication.

The ultrasound probe 1 comprises a transducer array 11 and a transmission and reception circuit 12 connected to the transducer array 11.

The apparatus main body 2 comprises an image generation unit 21 connected to the transmission and reception circuit 12. In the apparatus main body 2, a display control unit 22 and a monitor 23 are sequentially connected to the image generation unit 21. In addition, a contour extraction unit 24, a cross-sectional area calculation unit 25, and a cross-sectional area change curve generation unit 26 are sequentially connected to the image generation unit 21. A cross-sectional area change prediction curve generation unit 27 is connected to the cross-sectional area change curve generation unit 26. An error detection unit 28 is connected to the cross-sectional area change curve generation unit 26 and the cross-sectional area change prediction curve generation unit 27. In addition, a memory 29 is connected to the contour extraction unit 24, and an error correction unit 30 is connected to the memory 29. The error correction unit 30 is connected to the memory 29. The memory 29 is further connected to the cross-sectional area calculation unit 25. In addition, the cross-sectional area change curve generation unit 26, the cross-sectional area change prediction curve generation unit 27, the error detection unit 28, and the error correction unit 30 are connected to the display control unit 22. In addition, a main body control unit 31 is connected to the transmission and reception circuit 12, the image generation unit 21, the display control unit 22, the contour extraction unit 24, the cross-sectional area calculation unit 25, the cross-sectional area change curve generation unit 26, the cross-sectional area change prediction curve generation unit 27, the error detection unit 28, the memory 29, and the error correction unit 30. An input device 32 is connected to the main body control unit 31.

The transmission and reception circuit 12 and the image generation unit 21 constitute an image acquisition unit 33. In addition, the image generation unit 21, the display control unit 22, the contour extraction unit 24, the cross-sectional area calculation unit 25, the cross-sectional area change curve generation unit 26, the cross-sectional area change prediction curve generation unit 27, the error detection unit 28, the error correction unit 30, and the main body control unit 31 constitute a processor 34 for the apparatus main body 2.

The transducer array 11 of the ultrasound probe 1 includes a plurality of ultrasound transducers arranged one-dimensionally or two-dimensionally. In response to a drive signal supplied from the transmission and reception circuit 12, each of the ultrasound transducers transmits ultrasound and receives an ultrasound echo from a subject to output a signal based on the ultrasound echo. For example, each ultrasound transducer is configured by forming electrodes at both ends of a piezoelectric material consisting of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), and the like.

The image acquisition unit 33 configured by the transmission and reception circuit 12 and the image generation unit 21 acquires an ultrasound image of an inside of the subject by transmitting and receiving an ultrasound beam by using the ultrasound probe 1.

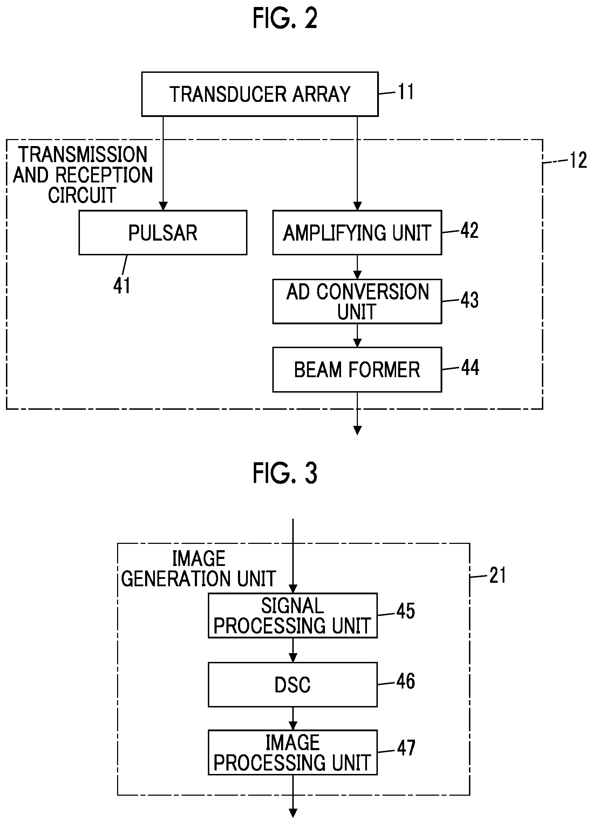

The transmission and reception circuit 12 transmits the ultrasound wave from the transducer array 11 and generates a sound ray signal based on a reception signal acquired by the transducer array 11, under the control of the main body control unit 31. As shown in Fig. 2, the transmission and reception circuit 12 includes a pulsar 41 connected to the transducer array 11, and an amplifying unit 42, an analog-to-digital (AD) conversion unit 43, and a beam former 44 that are sequentially connected in series to the transducer array 11.

The pulsar 41 includes, for example, a plurality of pulse generators, and adjusts an amount of delay of each of drive signals and supplies the drive signals to the plurality of ultrasound transducers such that ultrasound waves transmitted from the plurality of ultrasound transducers of the transducer array 11 form an ultrasound beam based on a transmission delay pattern selected according to a control signal from the main body control unit 31. In this way, in a case where a pulsed or continuous wave-like voltage is applied to the electrodes of the ultrasound transducer of the transducer array 11, the piezoelectric body expands and contracts to generate a pulsed or continuous wave-like ultrasound wave from each of the ultrasound transducers, whereby an ultrasound beam is formed from the combined wave of these ultrasound waves.

The transmitted ultrasound beam is, for example, reflected in a target such as a part of the subject and propagates toward the transducer array 11 of the ultrasound probe 1. The ultrasound echo propagating toward the transducer array 11 in this way is received by each of the ultrasound transducers constituting the transducer array 11. In this case, each of the ultrasound transducers constituting the transducer array 11 receives the propagating ultrasound echo to expand and contract to generate the reception signal, which is an electrical signal, and outputs these reception signals to the amplifying unit 42.

The amplifying unit 42 amplifies the signal input from each of the ultrasound transducers constituting the transducer array 11 and transmits the amplified signal to the AD conversion unit 43. The AD conversion unit 43 converts the signal transmitted from the amplifying unit 42 into digital reception data. The beam former 44 performs so-called reception focus processing by applying and adding the delay to each reception data received from the AD conversion unit 43. By this reception focus processing, each reception data converted by the AD conversion unit 43 is phase-added, and the sound ray signal in which the focus of the ultrasound echo is narrowed down is acquired.

As shown in Fig. 3, the image generation unit 21 has a configuration in which a signal processing unit 45, a digital scan converter (DSC) 46, and an image processing unit 47 are sequentially connected in series to each other.

The signal processing unit 45 generates a B-mode image signal, which is tomographic image information regarding tissues within the subject, by performing, on the sound ray signal received from the transmission and reception circuit 12, correction of the attenuation due to the distance according to the depth of the reflection position of the ultrasound wave using a sound velocity value set by the main body control unit 31 and then performing envelope detection processing.

The DSC 46 converts (raster-converts) the B-mode image signal generated by the signal processing unit 45 into the image signal in accordance with a normal television signal scanning method.

The image processing unit 47 performs various kinds of necessary image processing such as gradation processing on the B-mode image signal input from the DSC 46, and then sends the B-mode image signal to the display control unit 22 and the contour extraction unit 24. Hereinafter, the B-mode image signal that is image-processed by the image processing unit 47 will be referred to as an ultrasound image.

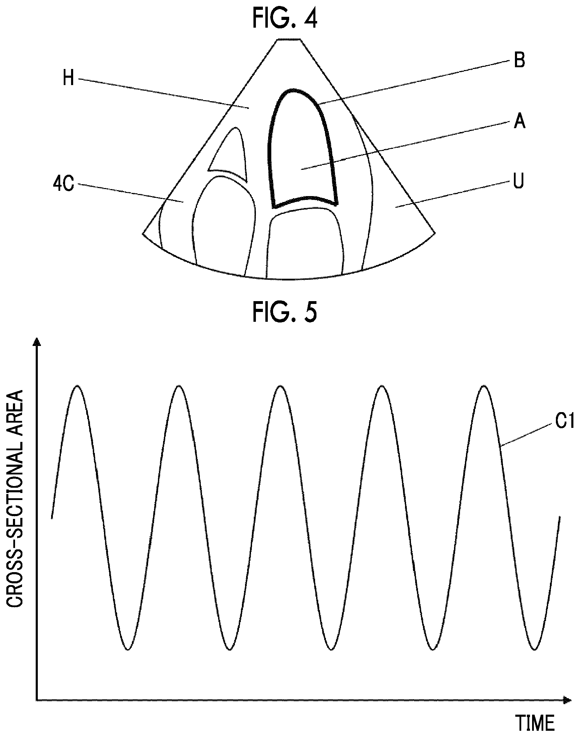

The ultrasound diagnostic apparatus according to the embodiment of the present invention is used to calculate an ejection fraction of the lumen of the heart of the subject, such as a left ventricular ejection fraction (LVEF). In order to calculate the ejection fraction of the lumen of the heart, the image acquisition unit 33 acquires the ultrasound images U of the plurality of frames representing a so-called apical four-chamber cross section 4C of the heart H as shown in Fig. 4 and the ultrasound images U of the plurality of frames representing a so-called apical two-chamber cross section of the heart H, which are not shown.

The display control unit 22 performs predetermined processing on the first ultrasound image U1, the second ultrasound image U2, and the like generated by the image generation unit 21 and displays the first ultrasound image U1, the second ultrasound image U2, and the like on the monitor 23, under the control of the main body control unit 31.

The monitor 23 is a monitor for displaying the first ultrasound image U1, the second ultrasound image U2, and the like under the control of the display control unit 22, and includes a display device such as a liquid crystal display (LCD), or an organic electroluminescence (EL) display.

The main body control unit 31 controls each unit of the apparatus main body 2 and the transmission and reception circuit 12 of the ultrasound probe 1 based on a control program or the like, which is stored in advance.

The input device 32 is an input device for the user to perform an input operation, and is configured by, for example, a device such as a keyboard, a mouse, a trackball, a touchpad, and a touch sensor disposed in a state of being superimposed on the monitor 23.

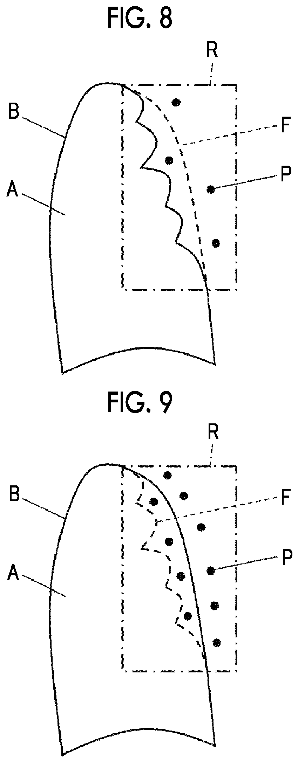

The contour extraction unit 24 performs segmentation on the ultrasound images U of the plurality of frames acquired by the image acquisition unit 33 to extract, for example, the contour B of the lumen A of the heart H for each frame as shown in Fig. 4. In Fig. 9, a left ventricle is shown as an example of the lumen A of the heart H.

The contour extraction unit 24 can extract the contour B of the lumen A of the heart H from the ultrasound image U by segmentation using a learning model in so-called machine learning in which a relationship between a plurality of ultrasound images including the lumen A of the heart H and the contour B of the lumen A is learned. In addition, the contour extraction unit 24 can also extract the contour B of the lumen A of the heart H from the ultrasound image U using a so-called template matching method of, for example, storing a plurality of template image data representing the lumen A of the heart H in advance and searching the ultrasound image U using the plurality of template image data.

The contour extraction unit 24 sends information on the contour B of the lumen A of the heart H extracted in this manner to the cross-sectional area calculation unit 25. In addition, the contour extraction unit 24 stores information on the contour B of the lumen A of the extracted heart H in the memory 29 together with the ultrasound image U from which the contour B is extracted.

The cross-sectional area calculation unit 25 calculates the cross-sectional area of the lumen A of the heart H for each frame based on the contour B extracted by the contour extraction unit 24. The cross-sectional area calculation unit 25 refers to the cross-sectional area of the lumen A of the heart H calculated here as the area of the lumen A of the heart H on the ultrasound image U. The cross-sectional area calculation unit 25 can calculate the cross-sectional area of the lumen A of the heart H by a known method, for example, calculating the total number of pixels in the extracted contour B.

The cross-sectional area change curve generation unit 26 generates a cross-sectional area change curve representing an actual temporal change of the cross-sectional area based on the cross-sectional area of the lumen A of the heart H calculated by the cross-sectional area calculation unit 25. For example, as schematically shown by a cross-sectional area change curve C1 of Fig. 5, the cross-sectional area of the lumen A of the heart H periodically changes over time in time series due to the beating of the heart H.

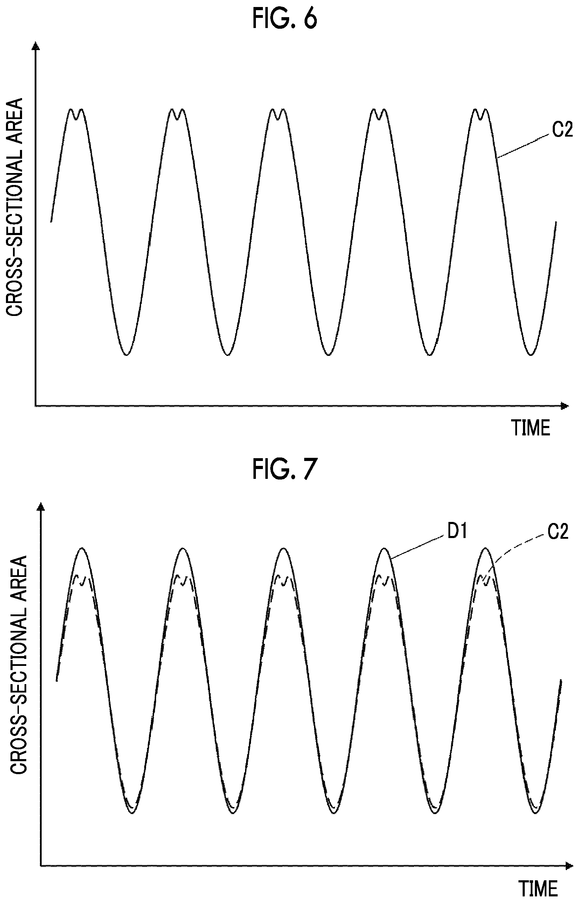

In a case where the segmentation is normally performed by the contour extraction unit 24 on all the acquired frames, that is, in a case where the original contour B of the lumen A of the heart H is normally extracted, for example, as schematically shown in Fig. 5, a cross-sectional area change curve C1 that normally represents a change in the beating of the heart H is obtained. On the other hand, in a case where an error occurs in the segmentation by the contour extraction unit 24 due to the presence of a so-called artifact in the ultrasound image U, and a contour B different from the original contour B of the lumen A of the heart H is extracted, for example, as schematically shown in Fig. 6, a partially distorted cross-sectional area change curve C2 may be obtained. In the example of Fig. 6, it is shown that a portion of the cross-sectional area change curve C2 corresponding to the maximal value in the cross-sectional area change curve C1 of Fig. 5 is distorted.

The cross-sectional area change prediction curve generation unit 27 generates, for example, a cross-sectional area change prediction curve D1 as shown in Fig. 7 based on the cross-sectional area change curve C1 or C2 generated by the cross-sectional area change curve generation unit 26. The cross-sectional area change prediction curve D1 is a curve representing a time-series change in the cross-sectional area of the lumen A of the heart H, which is predicted on the assumption that the segmentation is normally performed in all of the ultrasound images U of the plurality of frames. The cross-sectional area change prediction curve generation unit 27 can generate, for example, a curve obtained by approximating the cross-sectional area change curve C1 or C2 to a so-called Bézier curve as the cross-sectional area change prediction curve D1 using, for example, a so-called least squares method. In the example of Fig. 7, a cross-sectional area change prediction curve D1 obtained by approximating the cross-sectional area change curve C2 in which a part is distorted with a Bézier curve is shown. In this example, the cross-sectional area change curve C2 and the cross-sectional area change prediction curve D1 do not intersect with each other in a portion including the maximal value of the cross-sectional area change prediction curve D1, and are separated from each other.

The error detection unit 28 detects an error of the segmentation performed by the contour extraction unit 24 by comparing the cross-sectional area change curve C1 or C2 generated by the cross-sectional area change curve generation unit 26 with the predicted cross-sectional area change prediction curve D1. The error detection unit 28 can detect, for example, that the cross-sectional area change curve C1 or C2 and the cross-sectional area change prediction curve D1 are at least partially separated from each other as the error of the segmentation. For example, in the example of Fig. 7, since the cross-sectional area change curve C2 and the cross-sectional area change prediction curve D1 are separated from each other in a portion including the maximal value of the cross-sectional area change prediction curve D1, this is detected as an error of the segmentation.

As described above, since the error detection unit 28 automatically detects the error of the segmentation by comparing the cross-sectional area change curve C1 or C2 with the cross-sectional area change prediction curve D1, the user of the ultrasound diagnostic apparatus does not need to check the ultrasound images U of the plurality of frames to find the error of the segmentation, and the error of the segmentation can be easily found in a short time.

The error detection unit 28 can notify the user of the detection result of the error by displaying, for example, a message such as "an error has occurred in the segmentation" on the monitor 23 together with the cross-sectional area change curve C2 and the cross-sectional area change prediction curve D1 as shown in Fig. 7. The user can check only the ultrasound image U of the required frame in order to correct the error in the segmentation by confirming the notification by the error detection unit 28.

The memory 29 stores the contour B of the lumen A of the heart H extracted by the contour extraction unit 24 and the contour information deformed by the error correction unit 30, which will be described later, under the control of the main body control unit 31.

Here, as the memory 29, for example, a recording medium such as a flash memory, a hard disk drive (HDD), a solid-state drive (SSD), a flexible disk (FD), a magneto-optical disk (MO disk), a magnetic tape (MT), a random-access memory (RAM), a compact disc (CD), a digital versatile disc (DVD), a secure digital card (SD card), or a universal serial bus memory (USB memory) can be used.

The error correction unit 30 corrects the error of the segmentation performed by the contour extraction unit 24 by deforming the contour B of the lumen A of the heart H extracted by the contour extraction unit 24 based on the input operation of the user via the input device 32. For example, as shown in Fig. 8, the error correction unit 30 can dispose a plurality of control points P that are used to deform the contour B in the image region R on the ultrasound image U specified by the user via the input device 32 and that can be moved by the user, on the ultrasound image U, and can deform the contour B in response to the movement of the plurality of control points P by the user. In this case, the error correction unit 30 can approximate the contour B in the image region R with a Bézier curve, for example, to dispose a plurality of control points of the approximated Bézier curve as a plurality of control points P.

In this case, for example, as shown in Fig. 8, the error correction unit 30 can set the number of the plurality of control points P by calculating the number of inflection points in the contour B extracted by the contour extraction unit 24 in the image region R specified by the user, detecting the edge F of the lumen A of the heart H in the image region R, calculating the number of inflection points in the edge F, and comparing the number of inflection points in the contour B with the number of inflection points in the edge F. Here, for example, the error correction unit 30 can create a plurality of brightness profiles along the scanning line in the ultrasound image U, and detect a portion where the value of the brightness is equal to or greater than a certain brightness threshold value in each of the brightness profiles as the edge F.

For example, as shown in Fig. 8, in a case where the number of inflection points in the detected edge F is smaller than the number of inflection points in the contour B, the error correction unit 30 can set a predetermined first number of control points P. In addition, for example, as shown in Fig. 9, in a case where the number of inflection points in the detected edge F is larger than the number of inflection points in the contour B, the error correction unit 30 can set the control points P of a predetermined second number larger than a predetermined first number.

For example, the error correction unit 30 can also set the number of control points P in accordance with a difference between the number of inflection points in the detected edge F and the number of inflection points in the contour B.

The information on the contour B after being corrected by the error correction unit 30 is stored in the memory 29. The information on the corrected contour B is read out from the memory 29 and sent to the cross-sectional area calculation unit 25. The cross-sectional area calculation unit 25 recalculates the cross-sectional area of the lumen A of the heart H based on the corrected contour B. The cross-sectional area change curve generation unit 26 updates the cross-sectional area change curve C2 by using the value of the cross-sectional area of the lumen A of the heart H after the recalculation instead of the cross-sectional area of the lumen A of the heart H before the recalculation. The cross-sectional area change prediction curve generation unit 27 regenerates the cross-sectional area change prediction curve D1 based on the updated cross-sectional area change curve C2. The error detection unit 28 performs processing of detecting the error of the segmentation by comparing the cross-sectional area change curve C2 after the update with the cross-sectional area change prediction curve D1 after the regeneration.

The user deforms the contour B of the lumen A of the heart H via the input device 32, for example, until it is determined that the error of the segmentation is appropriately corrected, while referring to the cross-sectional area change curve C2 after the update, the cross-sectional area change prediction curve D1 after the regeneration, and the detection result of the error of the segmentation based on these.

The processor 34 including the image generation unit 21, the display control unit 22, the contour extraction unit 24, the cross-sectional area calculation unit 25, the cross-sectional area change curve generation unit 26, the cross-sectional area change prediction curve generation unit 27, the error detection unit 28, the error correction unit 30, and the main body control unit 31 may be configured by one or a plurality of hardware, and the type of hardware is not limited. For example, the processor can be configured by a programmable logic device such as a central processing unit (CPU), a micro processing unit (MPU), or a field programmable gate array (FPGA), a dedicated circuit for executing specific processing such as an application specific integrated circuit (ASIC), or hardware such as a graphic processing unit (GPU) or a neural processing unit (NPU). In addition, the processor has each unit or each means that executes various types of processing in the present embodiment. In addition, the types of hardware may be a combination of different types of hardware. In a case where a plurality of hardware are configured to execute one or a plurality of processes of a certain processor, the plurality of hardware may be present in devices physically separated from each other, or may be present in the same device. In addition, in any of the embodiments, the order of each processing by the processor is not limited to the above order, and may be appropriately changed. The hardware is configured by an electric circuit (circuitry) in which circuit elements such as semiconductor elements are combined.

Further, the present embodiment may be realized by hardware, software, firmware, microcode, or a combination thereof. Software, firmware, and microcode are configured by programs. In addition, the program may be, for example, a program module group, and each function thereof may be realized by a processor configured to execute each function. The program may be a program code or a plurality of code segments stored in one or a plurality of non-transitory computer-readable media (for example, a storage medium or other storage). The program may be stored in a plurality of non-transitory computer-readable media existing in devices physically separated from each other. The program code or the code segment can represent any combination of a procedure, a function, a subprogram, a routine, a subroutine, a module, a software package, a class, or an instruction, a data structure, or a program statement. The program code or the code segment may be connected to another code segment or a hardware circuit by transmitting and receiving information, data, an argument, a parameter, or a content of a memory.

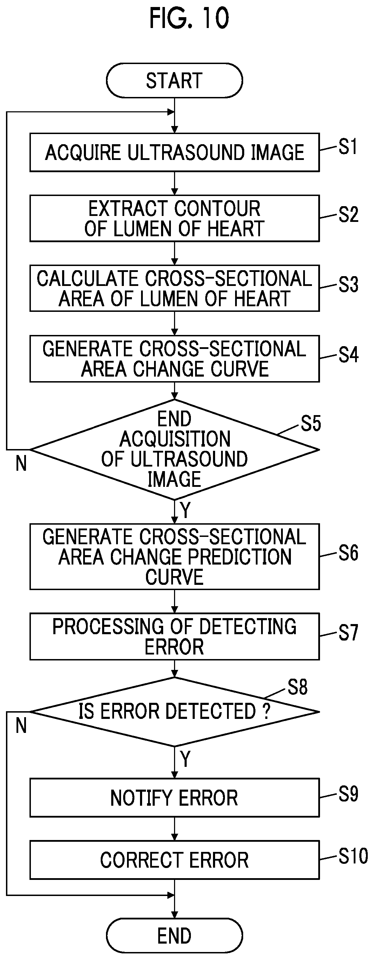

Next, an operation of the ultrasound diagnostic apparatus according to the embodiment will be described with reference to a flowchart shown in Fig. 10.

In step S1, the image acquisition unit 33 generates the ultrasound image U obtained by imaging the lumen A of the heart H. In this case, under the control of the main body control unit 31, the transmission and reception of the ultrasound from the plurality of transducers of the transducer array 11 are started in accordance with the drive signal from the pulsar 41 of the transmission and reception circuit 12 of the ultrasound probe 1, the ultrasound echo from the subject is received by the plurality of transducers of the transducer array 11, and the reception signal as the analog signal is output to the amplifying unit 42, is amplified, and then is subjected to the AD conversion via the AD conversion unit 43 to acquire the reception data.

The reception focus processing is performed on the reception data by the beam former 44, and the sound ray signal generated by the reception focusing processing is sent to the image generation unit 21 of the apparatus main body 2, thereby the ultrasound image U representing the lumen A of the heart H of the subject is generated by the image generation unit 21. In this case, the signal processing unit 45 of the image generation unit 21 performs the correction of the attenuation in accordance with the depth of the reflection position of the ultrasound and the envelope detection processing on the sound ray signal, the DSC 46 performs the conversion into the image signal in accordance with the normal television signal scanning method, and the image processing unit 47 performs various types of necessary image processing such as gradation processing. The first ultrasound image U1 generated in step S1 in this manner is displayed on the monitor 23 via the display control unit 22 and is sent to the contour extraction unit 24.

In step S2, the contour extraction unit 24 extracts the contour B of the lumen A of the heart H from the ultrasound image U acquired in step S1. The contour extraction unit 24 can extract the contour B of the lumen A of the heart H by using, for example, a trained model in machine learning in which a relationship between a large number of ultrasound images U and the contour B of the lumen A of the heart H shown in the ultrasound images U is learned.

The contour extraction unit 24 stores the information on the extracted contour B in the memory 29 in association with the ultrasound image U from which the contour B is extracted.

In step S3, the cross-sectional area calculation unit 25 calculates the cross-sectional area of the lumen A of the heart H based on the contour B of the lumen A of the heart H extracted in step S2. The cross-sectional area calculation unit 25 can calculate the cross-sectional area of the lumen A of the heart H, for example, by calculating the total number of pixels in the region surrounded by the contour B in the ultrasound image U.

In step S4, the cross-sectional area change curve generation unit 26 generates a cross-sectional area change curve C1 or C2 shown in Figs. 5 and 6 by plotting the value of the cross-sectional area of the lumen A of the heart H calculated in step S3 along the time axis.

In step S5, the main body control unit 31 determines whether to end the acquisition of the ultrasound image U. The main body control unit 31 can determine to end the acquisition of the ultrasound image U in a case where an instruction to end the capturing of the ultrasound image U is input by the user via, for example, the input device 32, and can determine to continue the acquisition of the ultrasound image U in a case where there is no particular instruction from the user via the input device 32.

In a case where the main body control unit 31 determines to continue the acquisition of the ultrasound image U in step S5, the process returns to step S1, and the ultrasound image U of the new frame is acquired. In step S2, the contour B of the lumen A of the heart H included in the ultrasound image U is extracted, and the cross-sectional area of the lumen A of the heart H is calculated in step S3 based on the contour B. Further, in step S4, the cross-sectional area of the lumen A of the heart H newly calculated in step S4 is additionally plotted on the cross-sectional area change curve C1 or C2 generated in the previous step S4 along the time axis, and the cross-sectional area change curve C1 or C2 is updated. By repeating the processing of step S1 to step S5 in this way, the cross-sectional area change curve C1 or C2 shown in Figs. 5 and 6, which indicates that the value of the cross-sectional area actually changes periodically, is obtained. In a case where it is determined in step S5 to end the acquisition of the ultrasound image U, the process proceeds to step S6.

In step S6, the cross-sectional area change prediction curve generation unit 27 generates, for example, a cross-sectional area change prediction curve D1 predicted on the assumption that the segmentation is normally performed in all the ultrasound images U of the plurality of frames, as shown in Fig. 7, based on the cross-sectional area change curve C1 or C2 generated in the last step S4. The cross-sectional area change prediction curve generation unit 27 can generate, as the cross-sectional area change prediction curve D1, a curve obtained by approximating the cross-sectional area change curve C1 or C2 with a Bézier curve using, for example, a method such as a least squares method.

In step S7, the error detection unit 28 performs processing of detecting the error of the segmentation performed in step S2 in any of the plurality of frames by comparing the cross-sectional area change curve C1 or C2 generated in the last step S4 with the cross-sectional area change prediction curve D1 generated in step S6. For example, as shown in Fig. 7, the error detection unit 28 can detect that the cross-sectional area change curve C2 and the cross-sectional area change prediction curve D1 are separated from each other as an error of the segmentation in a case where a part of the waveform is distorted as in the cross-sectional area change curve C2.

As described above, since the error detection unit 28 automatically detects the error of the segmentation by comparing the cross-sectional area change curve C1 or C2 with the cross-sectional area change prediction curve D1, the user of the ultrasound diagnostic apparatus does not need to check the ultrasound images U of the plurality of frames to find the error of the segmentation, and the error of the segmentation can be easily found in a short time.

In step S8, the main body control unit 31 determines whether or not an error in the segmentation is detected with reference to the result of the error detection processing in step S7. In a case where it is determined that an error in the segmentation is detected, the process proceeds to step S9.

In step S9, the error detection unit 28 notifies the user that the error in the segmentation is detected in step S7. The error detection unit 28 can notify the user of the error by displaying, for example, a message such as "segmentation error has been detected" and the cross-sectional area change curve C2 and the cross-sectional area change prediction curve D1 as shown in Fig. 7 together on the monitor 23.

In step S10, the error correction unit 30 corrects the error of the segmentation by deforming the contour B of the lumen A of the heart H in the ultrasound image U of the frame in which the error of the segmentation is detected, based on the input operation of the user via the input device 32. In this case, the user checks the notification result of the error in step S9, selects the ultrasound image U of the frame in which the error of the segmentation is detected from the ultrasound images U of the plurality of frames, and inputs an instruction to deform the contour B extracted in the ultrasound image U of the frame.

For example, as shown in Figs. 8 and 9, the error correction unit 30 can set a plurality of control points P that are movable by the user on the ultrasound image U in order to deform the extracted contour B in the image region R specified by the user, and deform the contour B in response to the movement of the control points P by the user. In addition, the error correction unit 30 can detect the edge F of the lumen A of the heart H in the image region R specified by the user, and can set the number of the plurality of control points P by comparing the number of the inflection points of the detected edge F with the number of the inflection points of the contour B in the image region R with each other.

For example, in a case where the number of inflection points of the edge F is smaller than the number of inflection points of the contour B, that is, in a case where the edge F is smoother than the contour B as shown in Fig. 8, the error correction unit 30 can dispose a predetermined first number of control points P on the ultrasound image U in order to deform the contour B to be smooth. In addition, for example, in a case where the number of inflection points of the edge F is larger than the number of inflection points of the contour B as shown in Fig. 9, that is, in a case where the edge F includes more unevenness than the contour B, the error correction unit 30 can dispose the control points P of a predetermined second number larger than the predetermined first number on the ultrasound image U in order to deform the contour B to include more unevenness.

In a case where the contour B of the lumen A of the heart H is deformed by the error correction unit 30, information on the deformed contour B is stored in the memory 29. Then, the information on the deformed contour B is read out from the memory 29 and sent to the cross-sectional area calculation unit 25. The cross-sectional area calculation unit 25 recalculates the cross-sectional area of the lumen A of the heart H based on the deformed contour B. The cross-sectional area change curve generation unit 26 updates the cross-sectional area change curve C2 by re-plotting the cross-sectional area of the lumen A of the heart H obtained by the recalculation, instead of the cross-sectional area calculated based on the contour B before the deformation. The cross-sectional area change prediction curve generation unit 27 regenerates the cross-sectional area change prediction curve D1 based on the updated cross-sectional area change curve C2. The user can deform the contour B while checking the ultrasound image U in which the updated cross-sectional area change curve C2, the regenerated cross-sectional area change prediction curve D1, and the contour B are being corrected, which are displayed on the monitor 23.

As described above, since the error correction unit 30 can automatically set the number of control points P by comparing the detected edge F with the contour B in the image region R, the extracted contour B can be easily deformed such that the contour B is closer to the original contour of the lumen A of the heart H based on the instruction of the user. The cross-sectional area calculated from the contour B finally obtained by being corrected by the error correction unit 30 is used, for example, to calculate the ejection fraction of the lumen A of the heart H.

In a case in which the processing of step S10 is completed in this manner, the operation of the ultrasound diagnostic apparatus according to the flowchart of Fig. 10 is completed.

In addition, for example, in a case where the cross-sectional area change curve C1 generated in the last step S4 does not include distortion in the waveform and all of the cross-sectional area change curve C1 and the cross-sectional area change prediction curve D1 overlap each other, and thus the error of the segmentation is not detected in step S7, it is determined in step S8 that the error of the segmentation is not detected. In this case, the processing of step S9 and step S10 is skipped, and the operation of the ultrasound diagnostic apparatus according to the flowchart of Fig. 10 is completed. In this case, the cross-sectional area corresponding to the maximal value and the minimal value in the cross-sectional area change curve C1 is used, for example, to calculate the ejection fraction of the lumen A of the heart H.

As described above, with the ultrasound diagnostic apparatus according to the embodiment of the present invention, the contour extraction unit 24 performs segmentation on the ultrasound images U of the plurality of frames to extract the contour B of the lumen A of the heart H for each frame, the cross-sectional area calculation unit 25 calculates the cross-sectional area of the lumen A of the heart H for each frame on the basis of the extracted contour B, the cross-sectional area change curve generation unit 26 generates the cross-sectional area change curve C1 or C2 representing the actual temporal change of the cross-sectional area on the basis of the calculated cross-sectional area, and the error detection unit 28 detects the error of the segmentation performed by the contour extraction unit 24 by comparing the cross-sectional area change curve C1 or C2 with the predicted cross-sectional area change prediction curve D1. Therefore, the segmentation result of the lumen A of the heart H can be easily corrected in a short time.

It should be noted that a case has been described in which the transmission and reception circuit 12 is provided in the ultrasound probe 1, but the transmission-and-reception circuit 12 may be provided in the apparatus main body 2.

Further, a case has been described in which the image generation unit 21 is provided in the apparatus main body 2, but the image generation unit 21 may be provided in the ultrasound probe 1.

The apparatus main body 2 may be a so-called stationary type, a portable type that is easy to carry, or a so-called handheld type that is configured by, for example, a smartphone or a tablet type computer. As described above, the type of the device constituting the apparatus main body 2 is not particularly limited.

In general, in a case of calculating the ejection fraction of the lumen A of the heart H, the ultrasound images U corresponding to the end-diastole and the end-systole of the heart H are often used. In the ultrasound image U corresponding to the end-diastole and the end-systole of the heart H, the cross-sectional area of the lumen A of the heart H has a maximal value or a minimal value in the cross-sectional area change curve C1 or C2. Therefore, for example, the error detection unit 28 can detect an error only in a case where the portions corresponding to the maximal value and the minimal value of the cross-sectional area change prediction curve D1 do not overlap with the cross-sectional area change curve C2 and are separated from each other, and can notify the user of the error only in this case. As a result, the user can easily understand that the error of the segmentation is detected in the ultrasound image U of the frame used for calculating the ejection fraction of the lumen A of the heart H.

In addition, although it has been described that the error detection unit 28 notifies the error of the segmentation by displaying the message on the monitor 23, the notification method is not limited to the display of the message. For example, in a case where the ultrasound diagnostic apparatus comprises a speaker (not shown), the error detection unit 28 can also notify the user by a sound emitted from the speaker. In addition, in a case where the ultrasound diagnostic apparatus comprises a light emitting unit (not shown), the error detection unit 28 can also notify the user of the error by light emitted from the light emitting unit.

### Explanation of References

1: ultrasound probe
2: apparatus main body
11: transducer array
12: transmission and reception circuit
21: image generation unit
22: display control unit
23: monitor
24: contour extraction unit
25: cross-sectional area calculation unit
26: cross-sectional area change curve generation unit
27: cross-sectional area change prediction curve generation unit
28: error detection unit
29: memory
30: error correction unit
31: main body control unit
32: input device
33: image acquisition unit
34: processor
41: pulsar
42: amplifying unit
43: AD conversion unit
44: beam former
45: signal processing unit
46: DSC
47: image processing unit
4C: apical four-chamber cross section
A: lumen
B: contour
C1, C2: cross-sectional area change curve
D1: cross-sectional area change prediction curve
F: edge
H: heart
P: control point
R: image region
U: ultrasound image

## Claims

1. An ultrasound diagnostic apparatus comprising:
an image acquisition unit (33) that acquires ultrasound images of a plurality of frames that are continuous in time series and in which a heart of a subject is imaged;
a contour extraction unit (24) that performs segmentation on the ultrasound images of the plurality of frames to extract a contour of a lumen of the heart for each frame;
a cross-sectional area calculation unit (25) that calculates a cross-sectional area of the lumen of the heart for each frame based on the contour extracted by the contour extraction unit (24); and **characterized by**
a cross-sectional area change curve generation unit (26) that generates a cross-sectional area change curve representing an actual temporal change of the cross-sectional area based on the cross-sectional area calculated by the cross-sectional area calculation unit (25); and
an error detection unit (28) that detects an error of the segmentation performed by the contour extraction unit by comparing the cross-sectional area change curve generated by the cross-sectional area change curve generation unit (26) with a predicted cross-sectional area change prediction curve.

2. The ultrasound diagnostic apparatus according to claim 1,
wherein the error detection unit (28) notifies a user of a detection result of the error.

3. The ultrasound diagnostic apparatus according to claim 1 or 2,
wherein the error detection unit (28) detects that the cross-sectional area change curve and the cross-sectional area change prediction curve are at least partially separated from each other as the error.

4. The ultrasound diagnostic apparatus according to any one of claims 1 to 3, further comprising:
a prediction curve generation unit (27) that generates the cross-sectional area change prediction curve based on the cross-sectional area change curve generated by the cross-sectional area change curve generation unit (26).

5. The ultrasound diagnostic apparatus according to any one of claims 1 to 4, further comprising:
an error correction unit (30) that corrects the error of the segmentation performed by the contour extraction unit (24).

6. The ultrasound diagnostic apparatus according to claim 5,
wherein the error correction unit (30)
sets a plurality of control points operable by a user on the contour extracted by the contour extraction unit, and
corrects the error of the segmentation by deforming the contour based on movement of at least one of the plurality of control points by the user.

7. The ultrasound diagnostic apparatus according to either claim 5 or claim 6,
wherein the error correction unit (30)
calculates the number of inflection points in the contour extracted by the contour extraction unit (24) in an image region specified by the user,
detects an edge of the lumen of the heart in the image region and calculates the number of inflection points in the edge, and
sets the number of the plurality of control points by comparing the number of inflection points in the contour with the number of inflection points in the edge.

8. A control method of an ultrasound diagnostic apparatus, the control method comprising:
acquiring ultrasound images of a plurality of frames that are continuous in time series and in which a heart of a subject is imaged;
performing segmentation on the ultrasound images of the plurality of frames to extract a contour of a lumen of the heart for each frame;
calculating a cross-sectional area of the lumen of the heart for each frame based on the extracted contour; and **characterized by**
generating a cross-sectional area change curve representing an actual temporal change of the cross-sectional area based on the calculated cross-sectional area; and
detecting an error of the segmentation by comparing the generated cross-sectional area change curve with a predicted cross-sectional area change prediction curve.

## Patentansprüche

1. Ultraschalldiagnosevorrichtung, umfassend:
eine Bilderfassungseinheit (33), die Ultraschallbilder von mehreren Einzelbildern, die in Zeitreihe kontinuierlich sind und in denen ein Herz einer Untersuchungsperson abgebildet ist, erfasst;
eine Konturextraktionseinheit (24), die Segmentierung an den Ultraschallbildern der mehreren Einzelbilder durchführt, um eine Kontur eines Lumens des Herzens für jedes Einzelbild zu extrahieren;
eine Querschnittsflächen-Berechnungseinheit (25), die eine Querschnittsfläche des Lumens des Herzens für jedes Einzelbild auf der Grundlage der von der Konturextraktionseinheit (24) extrahierten Kontur berechnet; und **gekennzeichnet durch**
eine Querschnittsflächen-Änderungskurven-Erzeugungseinheit (26), die eine Querschnittsflächen-Änderungskurve, die eine tatsächliche zeitliche Änderung der Querschnittsfläche darstellt, auf der Grundlage der von der Querschnittsflächen-Berechnungseinheit (25) berechneten Querschnittsfläche erzeugt; und
eine Fehlerdetektionseinheit (28), die einen Fehler der von der Konturextraktionseinheit durchgeführten Segmentierung detektiert, indem sie die von der Querschnittsflächen-Änderungskurven-Erzeugungseinheit (26) erzeugte Querschnittsflächen-Änderungskurve mit einer vorhergesagten Querschnittsflächen-Änderungsvorhersagekurve vergleicht.

2. Ultraschalldiagnosevorrichtung nach Anspruch 1,
wobei die Fehlerdetektionseinheit (28) einen Benutzer über ein Detektionsergebnis des Fehlers benachrichtigt.

3. Ultraschalldiagnosevorrichtung nach Anspruch 1 oder 2,
wobei die Fehlerdetektionseinheit (28) als den Fehler detektiert, dass die Querschnittsflächen-Änderungskurve und die Querschnittsflächen-Änderungsvorhersagekurve mindestens teilweise voneinander getrennt sind.

4. Ultraschalldiagnosevorrichtung nach einem der Ansprüche 1 bis 3, ferner umfassend:
eine Vorhersagekurven-Erzeugungseinheit (27), die die Querschnittsflächen-Änderungsvorhersagekurve auf der Grundlage der von der Querschnittsflächen - Änderungskurven-Erzeugungseinheit (26) erzeugten Querschnittsflächen-Änderungskurve erzeugt.

5. Ultraschalldiagnosevorrichtung nach einem der Ansprüche 1 bis 4, ferner umfassend:
eine Fehlerkorrektureinheit (30), die den Fehler der von der Konturextraktionseinheit (24) durchgeführten Segmentierung korrigiert.

6. Ultraschalldiagnosevorrichtung nach Anspruch 5,
wobei die Fehlerkorrektureinheit (30)
mehrere von einem Benutzer bedienbare Steuerpunkte auf der von der Konturextraktionseinheit extrahierten Kontur einstellt, und
den Fehler der Segmentierung durch Verformen der Kontur auf der Grundlage von Bewegung von mindestens einem der mehreren Steuerpunkte durch den Benutzer korrigiert.

7. Ultraschalldiagnosevorrichtung nach Anspruch 5 oder Anspruch 6,
wobei die Fehlerkorrektureinheit (30)
die Anzahl an Wendepunkten bei der von der Konturextraktionseinheit (24) in einem von dem Benutzer spezifizierten Bildbereich extrahierten Kontur berechnet,
einen Rand des Lumens des Herzens in dem Bildbereich detektiert und die Anzahl an Wendepunkten bei dem Rand berechnet, und
die Anzahl der mehreren Steuerpunkte durch Vergleichen der Anzahl an Wendepunkten bei der Kontur mit der Anzahl an Wendepunkten bei dem Rand einstellt.

8. Steuerverfahren einer Ultraschalldiagnosevorrichtung, wobei das Steuerverfahren umfasst:
Erfassen von Ultraschallbildern von mehreren Einzelbildern, die in Zeitreihe kontinuierlich sind und in denen ein Herz einer Untersuchungsperson abgebildet ist;
Durchführen von Segmentierung an den Ultraschallbildern der mehreren Einzelbilder, um eine Kontur eines Lumens des Herzens für jedes Einzelbild zu extrahieren;
Berechnen einer Querschnittsfläche des Lumens des Herzens für jedes Einzelbild auf der Grundlage der extrahierten Kontur; und **gekennzeichnet durch**
Erzeugen einer Querschnittsflächen-Änderungskurve, die eine tatsächliche zeitliche Änderung der Querschnittsfläche darstellt, auf der Grundlage der berechneten Querschnittsfläche; und
Detektieren eines Fehlers der Segmentierung durch Vergleichen der erzeugten Querschnittsflächen-Änderungskurve mit einer vorhergesagten Querschnittsflächen-Änderungsvorhersagekurve.

## Revendications

1. Appareil de diagnostic par ultrasons comprenant :
une unité d'acquisition d'images (33) qui acquiert des images ultrasonores d'une pluralité de trames qui sont continues en série temporelle et dans lesquelles un cœur d'un sujet est imagé ;
une unité d'extraction de contour (24) qui effectue une segmentation sur les images ultrasonores de la pluralité de trames pour extraire un contour d'un lumen du cœur pour chaque trame ;
une unité de calcul de surface de section transversale (25) qui calcule une surface de section transversale du lumen du cœur pour chaque trame sur la base du contour extrait par l'unité d'extraction de contour (24) ; et **caractérisé par**
une unité de génération de courbe de variation de surface de section transversale (26) qui génère une courbe de variation de surface de section transversale représentant une variation temporelle réelle de la surface de section transversale sur la base de la surface de section transversale calculée par l'unité de calcul de surface de section transversale (25) ; et
une unité de détection d'erreur (28) qui détecte une erreur de la segmentation effectuée par l'unité d'extraction de contour en comparant la courbe de variation de surface de section transversale générée par l'unité de génération de courbe de variation de surface de section transversale (26) avec une courbe de prédiction de variation de surface de section transversale prédite.

2. Appareil de diagnostic par ultrasons selon la revendication 1,
dans lequel l'unité de détection d'erreur (28) notifie à un utilisateur d'un résultat de détection de l'erreur.

3. Appareil de diagnostic par ultrasons selon la revendication 1 ou la revendication 2, dans lequel l'unité de détection d'erreur (28) détecte que la courbe de variation de surface de section transversale et la courbe de prédiction de variation de surface de section transversale sont au moins partiellement séparées l'une de l'autre comme l'erreur.

4. Appareil de diagnostic par ultrasons selon l'une quelconque des revendications 1 à 3, comprenant en outre :
une unité de génération de courbe de prédiction (27) qui génère la courbe de prédiction de variation de surface de section transversale sur la base de la courbe de variation de surface de section transversale générée par l'unité de génération de courbe de variation de surface de section transversale (26).

5. Appareil de diagnostic par ultrasons selon l'une quelconque des revendications 1 à 4, comprenant en outre :
une unité de correction d'erreur (30) qui corrige l'erreur de la segmentation effectuée par l'unité d'extraction de contour (24).

6. Appareil de diagnostic par ultrasons selon la revendication 5,
dans lequel l'unité de correction d'erreur (30)
définit une pluralité de points de contrôle manipulables par un utilisateur sur le contour extrait par l'unité d'extraction de contour, et
corrige l'erreur de la segmentation en déformant le contour sur la base de mouvement d'au moins l'un de la pluralité de points de contrôle par l'utilisateur.

7. Appareil de diagnostic par ultrasons selon la revendication 5 ou la revendication 6, dans lequel l'unité de correction d'erreur (30)
calcule le nombre de points d'inflexion dans le contour extrait par l'unité d'extraction de contour (24) dans une région d'image spécifiée par l'utilisateur,
détecte un bord du lumen du cœur dans la région d'image et calcule le nombre de points d'inflexion dans le bord, et
définit le nombre de la pluralité de points de contrôle en comparant le nombre de points d'inflexion dans le contour avec le nombre de points d'inflexion dans le bord.

8. Procédé de contrôle d'un appareil de diagnostic par ultrasons, le procédé de contrôle comprenant :
acquérir des images ultrasonores d'une pluralité de trames qui sont continues en série temporelle et dans lesquelles un cœur d'un sujet est imagé ;
effectuer une segmentation sur les images ultrasonores de la pluralité de trames pour extraire un contour d'un lumen du cœur pour chaque trame ;
calculer une surface de section transversale du lumen du cœur pour chaque trame sur la base du contour extrait ; et **caractérisé par**
générer une courbe de variation de surface de section transversale représentant une variation temporelle réelle de la surface de section transversale sur la base de la surface de section transversale calculée ; et
détecter une erreur de la segmentation en comparant la courbe de variation de surface de section transversale générée avec une courbe de prédiction de variation de surface de section transversale prédite.
